(19) Europäisches Patentamt

European Patent Office

Office européen des brevets

(11) EP 0 624 370 B1

(12) **EUROPÄISCHE PATENTSCHRIFT**

(45) Veröffentlichungstag und Bekanntmachung des Hinweises auf die Patenterteilung:
06.10.1999 Patentblatt 1999/40

(51) Int. Cl.⁶: **A61K 31/535**, A61K 9/20, A61K 47/34

(21) Anmeldenummer: 94106366.1

(22) Anmeldetag: 23.04.1994

(54) **Galenische Zubereitungen von Molsidomin**

Galenic compositions containing molsidomine

Compositions galéniques à base de molsidomine

(84) Benannte Vertragsstaaten:
AT BE CH DE ES FR IT LI NL

(30) Priorität: 11.05.1993 DE 4315664
11.05.1993 DE 9307100 U

(43) Veröffentlichungstag der Anmeldung:
17.11.1994 Patentblatt 1994/46

(73) Patentinhaber:
HOECHST AKTIENGESELLSCHAFT
65926 Frankfurt am Main (DE)

(72) Erfinder:
• Schraven, Eckhard, Dr.
D-60386 Frankfurt am Main (DE)
• Voegele, Dieter, Dr.
D-63486 Bruchköbel (DE)
• Baumann, Petra
D-63165 Mühlheim (DE)

(56) Entgegenhaltungen:
EP-A- 0 147 811          EP-A- 0 468 121

• CHEMICAL ABSTRACTS, vol. 119, no. 10, 6. September 1993, Columbus, Ohio, US; abstract no. 103090, & PHARM.IND., Bd.54, Nr.7, 1992 Seiten 630 - 638 K.THOMA ET AL. 'PHOTOINSTABILITY OF DRUGS.PART 6.PHOTOSTABILITY OF MOLSIDOMINE'

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft die Verwendung von Polyethylenglykolen zur Stabilisierung von Molsidomin in Tabletten sowie schnellzerfallende molsidominhaltige Tabletten, die durch einen Gehalt an Polyethylenglykol gekennzeichnet sind.

Molsidomin (N-Ethoxycarbonyl-3-morpholino-sydnonimin) ist ein wertvoller pharmakologischer Wirkstoff zur Behandlung koronarer Herzerkrankungen und seit vielen Jahren im Handel.

Nachteilig ist allerdings, daß Molsidomin relativ instabil, insbesondere photoinstabil, ist. Bereits 1971 konnten Glykolsäure, Chloressigsäure, Morpholin, Ammoniumchlorid, Ethanol, Kohlendioxid und Stickstoff als Zersetzungsprodukte identifiziert werden (Asahi et al, Chem. Pharm. Bull. 19, 1079 (1971)). Die genannte Instabilität führt zu erheblichen Gehaltsminderungen entsprechender galenischer Zubereitungen, die beispielsweise bei Tabletten nach einer 72-stündigen Bestrahlung mit Metallhalogenid-Strahlern (entspricht einer Lagerung von etwa 4 bis 6 Wochen am Tageslicht) bis zu 60 % betragen kann (Thoma, Kerker, Pharm.Ind. 54, Nr.7, 630 (1992)).

Es hat deshalb nicht an Versuchen gefehlt, Molsidomin in Tabletten, beispielsweise durch entsprechende Verpackungen oder den Zusatz von Stabilisatoren, wie Farbstoffen und UV-Absorbern, zu stabilisieren. In der EP-A-147811 wird die Photostabilisierung von Molsidomin durch bestimmte Hydroxyaromaten, zum Beispiel Troxerutin, beschrieben. Allerdings ist eine überzeugende und endgültige Lösung des Problems noch nicht vorgeschlagen worden.

Es ist bereits bekannt, daß Polyethylenglykole bei der Tablettenherstellung verwendet werden können. Sie wirken dabei als Binde-, Gleit-, Schmier- und Antibackmittel (Firmenbroschüre Polyethylenglykole der Hoechst AG, Seite 32). Ihr hohes Bindevermögen kann zu einer Verlängerung der Zerfallszeit der Tablette führen. Entsprechend werden Polyethylenglykole auch bereits als Steuerungsmittel zur Molsidominfreisetzung in retard-Tabletten eingesetzt.

Aus Thoma, Kerker, Pharm.Ind. 54, Nr.7, 630 (1992) ist bekannt, daß Molsidomin in Polyethylenglykol gelöst besonders photoinstabil ist. Zur Lösung des oben genannten Problems scheint Polyethylenglykol somit nicht geeignet zu sein. Um so überraschender ist es, daß nun gefunden wurde, daß Polyethylenglykole die Zersetzung von Molsidomin in Tabletten in erheblichem Ausmaß unterbinden.

Die vorliegende Erfindung betrifft somit die Verwendung von festen Polyethylenglykolen zur Stabilisierung von Molsidomin in Tabletten.

Die festen Polyethylenglykole werden dabei bevorzugt in Mengen von 1 bis 40 Gew.%, besonders bevorzugt 10 bis 30 Gew.%, bezogen auf das Tablettengewicht, eingesetzt.

Es kommen alle festen, pharmazeutisch unbedenklichen Polyethylenglykole in Frage. Ihr Molekulargewicht beträgt bevorzugt 1000 bis 35000, besonders bevorzugt 6000 bis 8000.

Die vorliegende Erfindung betrifft darüberhinaus schnellzerfallende molsidominhaltige Tabletten, dadurch gekennzeichnet, daß sie einen Gehalt an festem Polyethylenglykol aufweisen.

Schnellzerfallende Tabletten im Sinne der vorliegenden Erfindung sind Tabletten, die im Gegensatz zu retard-Formen schnell zerfallen und den Wirkstoff schnell abgeben. Insbesondere werden unter schnellzerfallenden Tabletten im Sinne der vorliegenden Erfindung solche verstanden, die im Zerfallstest gemäß DAB 10 (Kapitel V.5.1.1) innerhalb von 15 Minuten zerfallen sind.

Die erfindungsgemäßen Tabletten enthalten Molsidomin bevorzugt in Mengen von 0,5 bis 30 mg/Tablette, besonders bevorzugt in Mengen von 1 bis 15 mg/Tablette und ganz besonders bevorzugt in Mengen von 2 bis 8 mg/Tablette.

Die erfindungsgemäßen Tabletten enthalten bevorzugt die oben als bevorzugt bezeichneten Polyethylenglykole, bevorzugt in den oben als bevorzugt bezeichneten Mengen.

Entsprechendes gilt für die genannten besonders bevorzugten Polyethylenglykole und die besonders bevorzugten Mengen.

Daneben können die erfindungsgemäßen Tabletten die in schnellzerfallenden Tabletten üblichen Hilfs- und Trägerstoffe enthalten. Dazu gehören beispielsweise: Füllstoffe, wie Lactose, insbesondere Lactose-Monohydrat, Mannit, Stärke, insbesondere Maisstärke oder modifizierte Maisstärke, Cellulose oder Lactose-Cellulose-Kombinationen. Sprengmittel, wie quervernetztes Polyvinylpyrrolidon, Natriumstärkeglycolat oder quervernetzte Carboxymethylcellulose. Formtrennmittel, wie Magnesiumstearat, Natriumstearylfumarat oder hydriertes Rizinusöl. Bindemittel, wie Polyvinylpyrrolidon, mikrokristalline Cellulose oder Cellulosepulver. Weitere Stabilisatoren, wie beispielsweise die in EP-B 147811 und EP-B 206219 genannten, wobei Troxerutin bevorzugt ist.

Außerdem können die erfindungsgemäßen Tabletten auch noch übliche Geschmacks-, Süß- und Aromamittel enthalten.

Die erfindungsgemäßen Tabletten können nach üblichen, dem Fachmann bekannten Methoden zur Herstellung schnellzerfallender Tabletten hergestellt werden. Geeignete Methoden sind beispielsweise Feuchtgranulierung, Schmelzgranulierung, Trockengranulierung und Direktverpressung (siehe beispielsweise: Bauer, Frömming, Führer, Pharmazeutische Technologie, G. Thieme Verlag, Stuttgart 1986, Seite 363 ff).

Beispiel 1:

Durch Direktverpressung mit Tablettose® (Tablettose® ist ein eingetragenes Warenzeichen der Firma Meggle, Bundesrepublik Deutschland) hergestellte Tablette von 160 mg Gewicht, enthaltend:

| | |
|---|---|
| 2,0 mg | Molsidomin |
| 130,0 mg | Tablettose® |
| 3,2 mg | Polyplasdone® XL |
| 24,0 mg | Polyethylenglykol 6000 plv. |
| 0,8 mg | Magnesiumstearat |
| 160,0 mg | |
| (Polyplasdone® ist ein eingetragenes Warenzeichen der Firma GAF Chemicals, USA) | |

Beispiel 2:

Durch Trockengranulierung mit Lactose GK hergestellte Tablette von 160 mg Gewicht, enthaltend:

| | |
|---|---|
| 2,0 mg | Molsidomin |
| 130,0 mg | Lactose GK |
| 3,2 mg | Polyplasdone® XL |
| 24,0 mg | Polyethylenglykol 6000 plv. |
| 0,8 mg | Magnesiumstearat |
| 160,0 mg | |

Beispiel 3:

Durch Direktverpressung mit Lactose GK hergestellt Tablette von 160 mg Gewicht, zusammengesetzt wie die Tablette gemäß Beispiel 2.

Beispiel 4:

Durch Direktverpressung mit Lactose GK hergestellte Tablette von 320 mg Gewicht, enthaltend:

| | |
|---|---|
| 4,0 mg | Molsidomin |
| 260,0 mg | Lactose GK |
| 6,4 mg | Polyplasdone® XL |
| 48,0 mg | Polyethylenglykol 6000 plv. |
| 1,6 mg | Magnesiumstearat |
| 320,0 mg | |

Beispiel 5:

Durch Direktverpressung mit Lactose GK hergestellte Tablette von 160 mg Gewicht, enthaltend:

| | |
|---|---|
| 2,0 mg | Molsidomin |
| 2,0 mg | Troxerutin |
| 128,0 mg | Lactose GK |
| 3,2 mg | Polyplasdone® XL |
| 24,0 mg | Polyethylenglykol 6000 plv. |
| 0,8 mg | Magnesiumstearat |
| 160,0 mg | |

Beispiel 6:

Durch Direktverpressung mit Lactose GK hergestellte Tablette von 160 mg Gewicht, enthaltend:

| | |
|---|---|
| 2,0 mg | Molsidomin |
| 4,0 mg | Troxerutin |
| 126,0 mg | Lactose GK |
| 3,2 mg | Polyplasdone® XL |
| 24,0 mg | Polyethylenglykol 6000 plv. |
| 0,8 mg | Magnesiumstearat |
| 160,0 mg | |

Beispiel 7:

Durch Direktverpressung mit Lactose GK hergestellte Tablette von 160 mg Gewicht, enthaltend:

| | |
|---|---|
| 2,0 mg | Molsidomin |
| 8,0 mg | Troxerutin |
| 122,0 mg | Lactose GK |
| 3,2 mg | Polyplasdone® XL |
| 24,0 mg | Polyethylenglykol 6000 plv. |
| 0,8 mg | Magnesiumstearat |
| 160,0 mg | |

Die stabilisierende Wirkung des Polyglykols auf das Molsidomin wurde durch Bestimmung des Morpholingehalts der erfindungsgemäßen schnellzerfallenden Tabletten nach jeweils dreimonatiger Lagerung bei einer bestimmten Temperatur bzw. relativen Luftfeuchte in einer bestimmten Verpackung bestimmt. Dabei wurde der Morpholingehalt wie folgt bestimmt:

Die Tablette wird pulverisiert und mit Wasser extrahiert. Die so erhaltene wäßrige Lösung wird durch Flüssigchromatographie (LC) gemäß Pharm. Eur. 2 (V.6.20.4) analysiert. Es wird eine Zweisäulentechnik (Suppressor-Technik) angewendet, wobei eine Dionex-CS-10-Säule und als mobile Phase eine Lösung von 7 ml 37 % Salzsäure und 1000 mg DL-2,3-Diaminopropionsäure in 2000 ml doppelt destilliertem Wasser zur Anwendung kommt. Zur Regenerierung des Suppressors wird eine Lösung von 300 ml 40 %igem Tetrabutylammoniumhydroxid in 5000 ml doppelt destilliertem Wasser verwendet. Die Detektion erfolgt über eine Leitfähigkeitsmessung. Die Bestimmungsgrenze dieser Methode liegt bei 100 mg Morpholin/kg Molsidomin.

Als Vergleich wurden folgende Tabletten herangezogen:

A) Vergleichstablette von 160 mg Gewicht gemäß Stand der Technik ohne Polyglykolgehalt folgender Zusammensetzung:

| | |
|---|---|
| 2,0 mg | Molsidomin |
| 80,0 mg | Mannit |
| 44,3 mg | Maisstärke |
| 27,8 mg | Lactose 1$H_2O$ |
| 2,0 mg | Hydroxypropylmethylcellulose |
| 2,0 mg | Pfefferminzaroma |
| 1,2 mg | Magnesiumstearat |
| 0,5 mg | hochdisperses Siliciumdioxid |
| 0,2 mg | Gelborange S, wasserunlöslich |
| 160,0 mg | |

B) Vergleichstablette von 320 mg Gewicht gemäß Stand der Technik ohne Polyglykolgehalt folgender Zusammensetzung:

| | |
|---|---|
| 4,0 mg | Molsidomin |
| 160,0 mg | Mannit |
| 92,6 mg | Maisstärke |
| 56,0 mg | Lactose 1$H_2O$ |
| 4,0 mg | Hydroxypropylmethylcellulose |
| 2,4 mg | Magnesiumstearat |
| 1,0 mg | hochdisperses Siliciumdioxid |
| 320,0 mg | |

Es wurden folgende Ergebnisse erhalten:

| Tablette gemäß Beispiel | Packmittel | Morpholingehalt in mg/kg Molsidomin nach 3 Monaten bei | | | |
|---|---|---|---|---|---|
| | | +5°C | +25°C | +40°C | +40°C/75% relative Luftfeuchte |
| 1 | PVC grün | <100 | <100 | 170 | 290 |
| | PVC/PVDC grün | <100 | <100 | 100 | 250 |
| | Glasrundkörper braun | <100 | <100 | 100 | 190 |
| 2 | PVC grün | <100 | <100 | 110 | 340 |
| | PVC/PVDC grün | <100 | <100 | 150 | 420 |
| | Glasrundkörper braun | <100 | <100 | 200 | 130 |
| 3 | PVC grün | <100 | <100 | <100 | 210 |
| | PVC/PVDC grün | <100 | <100 | <100 | 230 |
| | Glasrundkörper braun | <100 | <100 | 230 | 150 |
| 4 | PVC grün | <100 | <100 | 300 | 370 |
| | PVC/PVDC grün | <100 | <100 | 190 | 350 |
| | Glasrundkörper braun | <100 | <100 | 190 | 170 |
| 5 | PCV grün | <100 | <100 | 150 | 150 |
| | PVC/PVDC grün | <100 | <100 | 130 | 230 |
| | Glasrundkörper braun | <100 | <100 | 160 | <100 |
| 6 | PVC grün | <100 | <100 | <100 | 100 |
| | PVC/PVDC grün | <100 | <100 | 100 | <100 |
| | Glasrundkörper braun | <100 | <100 | <100 | 100 |
| 7 | PVC grün | <100 | <100 | <100 | 160 |
| | PVC/PVDC grün | <100 | <100 | <100 | 100 |
| | Glasrundkörper braun | <100 | <100 | 100 | <100 |
| Vergleich A (Stand der Technik) | PVC grün | 810 | 1030 | 1040 | >2500 |
| | PVC/PVDC grün | 820 | 1030 | 1200 | >2500 |
| | Glasrundkörper braun | 820 | 350 | 2030 | >2500 |
| Vergleich B (Stand der Technik) | PVC grün | <100 | 100 | 510 | 2230 |
| | PVC/PVDC grün | 150 | 100 | 660 | >2500 |
| | Glasrundkörper braun | 100 | 110 | 1400 | 1980 |

**Patentansprüche**

1. Verwendung von festen Polyethylenglykolen zur Stabilisierung von Molsidomin in Tabletten.

2. Verwendung gemäß Anspruch 1, dadurch gekennzeichnet, daß feste Polyethylenglykole in Mengen von 1 bis 40 Gew.%, besonders bevorzugt 10 bis 30 Gew.%, bezogen auf das Tablettengewicht, eingesetzt werden.

3. Verwendung gemäß Anspruch 1 und/oder 2, dadurch gekennzeichnet, daß feste Polyethylenglykole vom Molekulargewicht 1000 bis 35000, besonders bevorzugt 6000 bis 8000, eingesetzt werden.

4. Schnellzerfallende molsidominhaltige Tabletten, dadurch gekennzeichnet, daß sie einen Gehalt an festem Polyethylenglykol aufweisen.

5. Tabletten gemäß Anspruch 4, dadurch gekennzeichnet, daß sie Molsidomin in Mengen von 0,5 bis 30 mg/Tablette, besonders bevorzugt 1 bis 15 mg/Tablette und ganz besonders bevorzugt 2 bis 8 mg/Tablette enthalten.

6. Tabletten gemäß Anspruch 4 und/oder 5, dadurch gekennzeichnet, daß sie feste Polyethylenglykole in Mengen von 1 bis 40 Gew.%, besonders bevorzugt 10 bis 30 Gew.%, bezogen auf das Tablettengewicht, enthalten.

7. Tabletten gemäß einem oder mehreren der Ansprüche 4 bis 6, dadurch gekennzeichnet, daß sie feste Polyethylenglykole vom Molekulargewicht 1000 bis 35000, besonders bevorzugt 6000 bis 8000, enthalten.

8. Tabletten gemäß einem oder mehreren der Ansprüche 4 bis 7, dadurch gekennzeichnet, daß sie übliche Hilfs- und Trägerstoffe enthalten.

9. Tabletten gemäß Anspruch 8, dadurch gekennzeichnet, daß sie Troxerutin enthalten.

**Claims**

1. The use of solid polyethylene glycols for the stabilization of molsidomine in tablets.

2. The use as claimed in claim 1, wherein solid polyethylene glycols are employed in amounts from 1 to 40% by weight, particularly preferably 10 to 30% by weight, based on the tablet weight.

3. The use as claimed in claim 1 and/or 2, wherein solid polyethylene glycols of molecular weight 1000 to 35,000, particularly preferably 6000 to 8000, are employed.

4. A rapidly disintegrating molsidomine-containing tablet, which contains solid polyethylene glycol.

5. The tablet as claimed in claim 4, which contains molsidomine in amounts of from 0.5 to 30 mg/tablet, particularly preferably 1 to 15 mg/tablet and very particularly preferably 2 to 8 mg/tablet.

6. A tablet as claimed in claim 4 and/or 5, which contains solid polyethylene glycols in amounts of from 1 to 40% by weight, particularly preferably 10 to 30% by weight, based on the tablet weight.

7. A tablet as claimed in one or more of claims 4 to 6, which contains solid polyethylene glycols of molecular weight 1000 to 35,000, particularly preferably 6000 to 8000.

8. A tablet as claimed in one or more of claims 4 to 7, which contains customary auxiliaries and excipients.

9. A tablet as claimed in claim 8, which contains troxerutin.

**Revendications**

1. Utilisation de polyéthylène glycols solides pour stabiliser la molsidomine dans des comprimés.

2. Utilisation selon la revendication 1, caractérisée en ce que les polyéthylène glycols sont utilisés en quantités allant de 1 à 40 % en poids, de façon particulièrement préférée de 10 à 30 % en poids, par rapport au poids des comprimés.

3. Utilisation selon la revendication 1 et/ou la revendication 2, caractérisée en ce que l'on utilise des polyéthylène glycols solides d'un poids moléculaire de 1 000 à 35 000, de façon particulièrement préférée de 6 000 à 8 000.

4. Comprimés à désagrégation rapide contenant de la molsidomine, caractérisés en ce qu'ils présentent une teneur en polyéthylène glycol solide.

5. Comprimés selon la revendication 4, caractérisés en ce qu'ils contiennent de la molsidomine en quantités allant de 0,5 à 30 mg/comprimé, de façon particulièrement préférée de 1 à 15 mg/comprimé, et de la façon la plus préférentielle de 2 à 8 mg/comprimé.

6. Comprimés selon la revendication 4 et/ou la revendication 5, caractérisés en ce qu'ils contiennent des polyéthylène glycols solides en quantités allant de 1 à 40 % en poids, de façon particulièrement préférée de 10 à 30 % en poids, par rapport au poids des comprimés.

7. Comprimés selon l'une ou plusieurs des revendications 4 à 6, caractérisés en ce qu'ils contiennent des polyéthylène glycols solides d'un poids moléculaire de 1 000 à 35 000, de façon particulièrement préférée de 6 000 à 8 000.

8. Comprimés selon l'une ou plusieurs des revendications 4 à 7, caractérisés en ce qu'ils contiennent des adjuvants et des vecteurs usuels.

9. Comprimés selon la revendication 8, caractérisés en ce qu'ils contiennent de la troxérutine.